# EUROPEAN PATENT SPECIFICATION

(11) **EP 2 126 053 B1**
(45) Date of publication and mention of the grant of the patent: **17.11.2010**
(21) Application number: 08717037.9
(22) Date of filing: 22.02.2008
(51) Int. Cl.: C12N 5/071, G01N 33/50

(54) **APOCRINE CELL LINE**
APOKRINE ZELLLINIE
LIGNÉE DE CELLULES APOCRINES

(30) Priority: 26.03.2007 GB 0705738
(43) Date of publication of application: 02.12.2009
(73) Proprietor: Unilever PLC, London EC4Y 0DY (GB); Unilever N.V., 3013 AL Rotterdam (NL)
(72) Inventor: BURRY, Jason, Shaun, Bebington, Wirral Merseyside CH63 3JW (GB); EVANS, Richard, Livesey, Bebington, Wirral Merseyside CH63 3JW (GB); HARKER, Mark, Bebington, Wirral Merseyside CH63 3JW (GB); KEALEY, George, Terence, Evelyn, Buckingham Buckinghamshire MK18 1EG (GB); MCDONALD, Donald, Farquhar, London Greater London N16 5HR (GB)
(74) Representative: Whaley, Christopher
(86) International application number: PCT/EP2008/052173
(87) International publication number: WO 2008/116713

(56) References cited:
- MARAS Z ET AL: "Cultivation of epithelia from the secretory coil of the ovine apocrine gland: evidence of secretory cell function and ductal morphogenesis in vitro." IN VITRO CELLULAR & DEVELOPMENTAL BIOLOGY. ANIMAL 1999 NOV-DEC, vol. 35, no. 10, November 1999 (1999-11), pages 606-611, XP008095807 ISSN: 1071-2690 cited in the application
- WICHE R ET AL: "Establishing of two in vitro models of epithelial cells from the apocrine secreting rat coagulating gland." ANDROLOGIA DEC 2003, vol. 35, no. 6, December 2003 (2003-12), pages 342-350, XP002493650 ISSN: 0303-4569 cited in the application
- WILSON S M ET AL: "Investigation of stimulus-secretion coupling in equine sweat gland epithelia using cell culture techniques." THE JOURNAL OF EXPERIMENTAL BIOLOGY OCT 1993, vol. 183, October 1993 (1993-10), pages 279-299, XP002493652 ISSN: 0022-0949
- LEE C M ET AL: "NCL-SG3: a human eccrine sweat gland cell line that retains the capacity for transepithelial ion transport." JOURNAL OF CELL SCIENCE FEB 1989, vol. 92 ( Pt 2), February 1989 (1989-02), pages 241-249, XP002493651 ISSN: 0021-9533

## Description

The present invention relates to an apocrine cell line exhibiting long-term proliferation derived from human apocrine glands, wherein long term indicates that cells retain their capability to proliferate after at least 30 sub-cultures (passages).

In addition to eccrine glands, apocrine glands secrete aqueous fluid containing various lipidic and aminoacid solutes which, when exposed on human skin to skin bacterial populations, are transformed to malodorous compounds, including steroids and short chain fatty acids. In other words, apocrine glands contribute to human sweating. Considerable research continues to be carried out to find means to counteract the generation of malodorous compounds on skin, but this is hampered by the absence of an apocrine cell line that closely mimics the functions of an apocrine gland *in situ*, i.e. in human skin. The development of the latter would be very valuable because it would offer one or more of the benefits mentioned herein. It would enable a greater number of *in vitro,* as opposed to *in vivo*, studies to be carried out. This would (i) assist in identifying the transport mechanisms within the apocrine gland that lead to sweating, (ii) facilitate the screening and identification of substances for reducing the formation of malodorous compounds, and (iii) help determine their effective concentration, i.e. the concentration required to act as a deodorant when applied topically to the body. The existence of a suitable cell line would also provide the opportunity to identify novel glandular functions other then sweating per se.

In common with attempts to sustain other secretory cells in long-term culture, primary cell lines obtained from apocrine cultures have not been passaged more than a few times before losing morphological, phenotypic and/or functional respects characteristic of an apocrine gland.

A paper by R Wiche et al in Andrologia 35, Dec (2003) pp342-350 entitled "Establishing of two in vitro models of epithelial cells from the apocrine secreting rat coagulating gland" relates to apocrine cells from rats, and does not disclose a human-derived apocrine cell line exhibiting long term proliferation. Furthermore, the cell markers used therein to demonstrate functionality are not necessarily relevant to the human apocrine model. Moreover, the authors seemingly did not seek to passage their cells after freezing and thawing, and thereby did not demonstrate their long term proliferation capability.

A paper by Z. Maras et al published in In Vitro Cell. Dev. Biol. Animal Vol 35, Nov-Dec (1999), pp 606-611 entitled "Cultivation of Epithelia from the Secretory Coil of the Ovine Apocrine Gland; Evidence of Secretory Cell Function and Ductal Morphogenesis in Vitro" relates to apocrine cells from sheep, and similarly does not disclose a human-derived apocrine cell line exhibiting long term proliferation.

A paper by Dieter C. Gruenert et al published in In Vitro Cell. Dev. Biol. Vol 26, April (1990), pp 411-416 entitled "Long Term Culture of Normal and Cystic Fibrosois Epithelial Cells grown under Serum-free Conditions" relates to eccrine cells instead of apocrine cells and therefore does not disclose a human-derived apocrine cell line exhibiting long term proliferation.

### Object of the present invention

It is an object of the present invention to provide an apocrine cell line derived from human a pocrine glands that mimics the sweating function of an apocrine gland, which can be maintained in culture through subcultures, and exhibits proliferation in the long term, wherein long term indicates that cells retain their capability to proliferate after at least 30 sub-cultures (passages).

### Brief summary of the present invention

According to the present invention there is provided a cultured apocrine cell line exhibiting long term proliferation derived from human apocrine glands.

Proliferation in the present context indicates that the cells retain the capability to divide when cultured in a suitable culture medium, but does not imply that the cell line is immortalised.

Long term, in the context of the present invention, indicates that cells retain their capability to proliferate after at least 30 sub-cultures (passages). In practice, when such long term proliferation has been achieved by a cell line, it can commonly exhibit proliferation through at least 100 or 1000 passages. Proliferation through such a large number of proliferations is indicative of indefinite proliferation, at least for practical purposes for cultured apocrine cell lines according to the present invention.

In particular, there is provided a human apocrine cell line ASG5 which has been deposited with the European Collection of Cell Cultures,(ECACC), Porton Down, Salisbury, SP4 0JG, England, under the depository number 07021301 and which exhibits at least long term proliferation.

### Detailed Description of preferred embodiments of the invention and morphology

This text describes the generation of a long term proliferative apocrine cell line, and subsequently, its characterisation and comparison with primary cultured apocrine cells via electrophysiological, molecular, and biochemical techniques. Particular interest is paid to steroid synthesis and the analysis of odour precursor compounds with express reference to the apocrine cell line ASG5, that has demonstrated at least long term proliferation.

### Isolation and primary culture of apocrine glands

An apocrine cell line that exhibited long term proliferation capability was obtained by the following method. Whole apocrine glands were isolated by shearing the axillary skin of women aged between 30 and 70, having previously obtained permission from the relevant ethical committee and informed consent of the subjects. Some apocrine sweat glands were fragmented by the shearing process, and cells could be cultured from those fragments. Microscopic observation and Neutral Red uptake experiments showed that the apocrine gland isolated by shearing consisted purely of coil separated from duct. On average between 10 and 100 apocrine coils were isolated from samples of axillary skin of about 50mm x 5mm.

The apocrine coils were then incubated using a modified method, proposed by Lee et al in J. Cell Sci. 83:103-118, 1986, in a Williams E medium (herein for short WEM) - containing collagenase type II (at 2 mg ml⁻¹) for 30 minutes at 37°C in 5% CO₂/air of 95% relative humidity. The coils were then washed in enzyme-free WEM supplemented with 10 ng ml⁻¹ epidermal growth factor (EGF), 10 µg ml⁻¹ insulin, 10 ng ml⁻¹ hydrocortisone, 10 µg ml⁻¹ holotransferrin, 1 mM L-glutamine, 100 U ml⁻¹ penicillin and 100 µg ml⁻¹ streptomycin.

Approximately 15 apocrine glands were plated out per 25cm² flasks in 1 ml of Mammary Epithelial Growth Medium (MEGM), supplemented with 10 ng ml⁻¹ EGF, 10 µg ml⁻¹ insulin, 0.5 µg ml⁻¹ hydrocortisone, 30 µg ml⁻¹ bovine pituitary extract, 50 µg ml⁻¹ gentamicin and 50 ng ml⁻¹ amphotericin. After 24hr incubation in a 95% air / 5% CO₂ humidified incubator at 37°C, a further 3ml MEGM was added to each flask and thereafter the medium was changed every 3 days.

### Generation and maintenance of a long term proliferating apocrine cell line (ASG5) - indicative of an indefinitely proliferating cell line

### Primary sweat gland isolation and culture

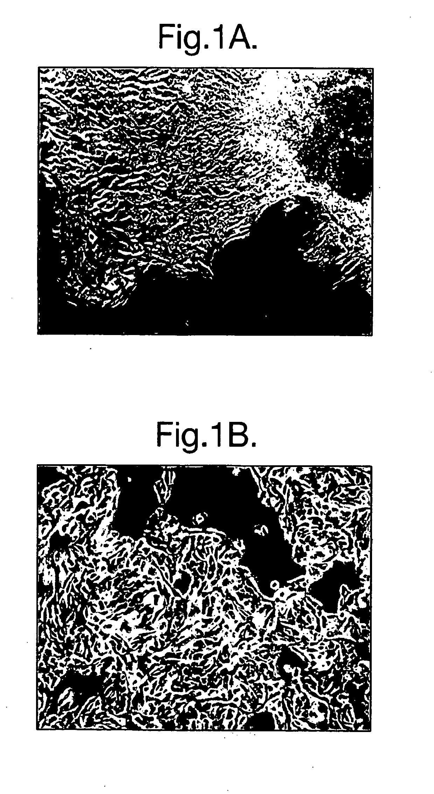

As indicated before, whole intact human apocrine sweat glands were isolated by shearing. Histological examination of apocrine glands revealed that shearing completely removed the short absorptive duct leaving only the secretory coil portion intact. Isolated glands were plated out in supplemented MEGM. Approximately 50% of glands plated out generated outgrowths, routinely observed after 2-3 days. Outgrowths continued to proliferate for up to 20 days and displayed typical 'cobblestone' epithelial cell morphology (as shown in Figure 1, which show phase contrast micrographs of (A) cells growing from an apocrine coil nine days after explantation, and (B) proliferating apocrine cells). At early stages of culture, before outgrowths were observed, a small number of cells with an elongated fibroblastoid morphology were often observed around explanted glands. However, these cells did not proliferate in MEGM.

A proliferating apocrine cell line was derived from primary cultures of apocrine secretory coils. After 7 days culture in MEGM, the potent phorbol ester, TPA (phorbol-12-myristate-13 acetate; ex Calbiochem, Nottingham, UK) was included in the medium at a concentration of 250 nM. It is an important feature of the process of obtaining a cell-line exhibiting long term proliferation capability that the culture medium contains an effective concentration of an ester of phorbol, phorbol being alternatively called 1,1a,1b,4,4a,7a,7b,8,9,9a-decahydro-4a,7b,9,9a-tetrahydroxy-3-(hydroxymethyl)-1,1,6,8-tetramethyl-5H-cyclopropa[3,4]benz[1,2-e]azulen-5-one. Commonly, the ester comprises at least aliphatic substituent particularly suitably at the 12 and/or 13 positions, such as a long chain alkyl (C₇ to C₂₄) and optionally also a short chain alkyl (C₂ to C₆). Accordingly, the method herein enables the preparation of an apocrine cell line exhibiting indefinite proliferation.

TPA induced premature multilayering 3-4 days after addition. However, the upper differentiated layers were removed by repeated washing to leave a proliferative monolayer of cells still attached to the flask. Cells of the proliferative layer were passaged into 96 well plates to generate clonal cell lines. Cells were maintained, passaged and continued to grow in MEGM containing TPA for approximately 2 months. After this period cells were cultured in MEGM alone and monitored for continued proliferation. Initially, many subclones continued to grow, but by the end of the next 10 passages, the majority had begun to senesce. One culture, in particular, ASG5, continued to grow and has now been maintained in culture, proliferating over more than 40 passages with no signs of a reduction in growth rate. This is indicative of exhibiting a proliferation capability indefinitely. This culture continues to proliferate in the absence of TPA, can survive cryopreservation and is considered to be stable. By comparison, primary apocrine sweat gland cultures grow for a maximum of 1 month and 4 passages and do not survive cryopreservation. The cell culture passaged herein exhibiting long term proliferation is morphologically similar to primary apocrine cultures, for example peak agonist responses. Such a cell culture is accordingly capable of being used to demonstrate whether and/or the extent to which a substance exhibits deodorising properties in vitro, i.e. is capable of acting as a personal deodorant.

Preparation of ASG5 apocrine cells for electrophysiology After 13-18 days, confluent ASG5 cultures were passaged onto Transwell collagen-coated permeable supports (0.33 cm² area, Costar, cat. no. 3495, High Wycombe, Bucks, UK). This involved incubation in 2% EDTA for 20 minutes, followed by trypsin (0.5%)-EDTA (0.2%) for approximately five minutes. 3 x10⁵ cells were transferred onto each Transwell supplemented with WEM. The Transwell supports were suspended in 24-well plates in 0.6 ml of the same medium. For primary cultures, only first passage cells were used in experiments. The medium bathing cultures on Transwells was changed every two days.

### Electrophysiology

Transepithelial resistance (TER) across epithelia on Transwells was monitored using 'chopstick' electrodes (WPI, Alresford, Hants, UK) connected to an ohmmeter. The TER was estimated by subtracting the measurement for a moistened filter blank. At peak TER, the Transwell supports were mounted in an Ussing Chamber. Epithelia were bathed in a modified Krebs buffer, oxygenated and maintained at 37°C, pH 7.4, with 95% O₂/5% CO₂. The buffer consisted of (mM): NaCl, 117; KCl, 4.7; CaCl₂, 1.5; MgSO₄, 1.2; NaHCO₃, 25; and glucose, 11.1. Voltage and current electrodes (calomel and silver/silver chloride respectively, ABB Kent Taylor, Stonehouse, Glos, UK), connected to a voltage/current clamp were extended into the bath via bridges containing agar dissolved in 3 M potassium chloride.

Voltage electrodes were balanced in the absence of a Transwell and fluid resistance between the voltage electrodes was compensated for by the injection of an arbitrary DC current. After mounting a Transwell the epithelium was short-circuited, the current allowed to stabilise, typically for 15 minutes. Given the rarity of apocrine material, drugs were typically added in succession as disclosed by Brayden et al (1988) J. Physiol, 405: 657-675, Pedersen et al (1992) Exp. Physiol, 77; 863-871, and Shen et al, (1994) Am. J. Physiol, 266: L493-501 and changes in short circuit current (I^{SC}) recorded. The duration of experiments was typically 15-60 min. TER was estimated using Ohm's Law, routinely by clamping the voltage at ± 10 mV and measuring the current required to achieve this. Unless otherwise stated all drugs listed in the legend were added to the basolateral side, except for amiloride that was added to the apical side. Amiloride is an inhibitor of the epithelial Na⁺ channel (ENaC) and the Na⁺/H⁺ exchanger.

### Modifications of Krebs Buffer

Chloride substitution experiments were carried out in Krebs buffer in which sodium gluconate was substituted for NaCl, potassium gluconate for KCl and CaSO₄ for CaCl₂. Glucose-free experiments were carried out with 11.1mM mannitol as a replacement to maintain osmolality. Experiments in which barium was used as an inhibitor of potassium channels were carried out in the absence of sulphate ions, such that MgCl₂ replaced MgSO₄.

### Basal transepithelial properties

Proliferating apocrine cultures were passaged onto permeable supports and transferred to an Ussing chamber at peak TER. The mean TER for such proliferating cultures was 395140Ωcm² (P<0.001). Resting short circuit current (I^{SC}) was 4.5±0.8 µA cm⁻² (P<0.001) for proliferating apocrine cultures. Resting transepithelial potential difference (TEPD) was 1.0 mV for proliferating apocrine cultures, apically negative.

### Effects of ion transport inhibitors on resting I^{SC} summarised in Table 1 below.

Resting I^{SC} in proliferating cultures was sensitive to 10 µM amiloride confirming that sodium reabsorption is a significant component of ion transport in cultured sweat gland epithelia. Apically applied amiloride (10 µM) reduced resting I^{SC} by 4.5±1.0 µA cm⁻² in proliferating apocrine cultures (n=20,P<0.001). Dose response curves reveal that the IC₅₀ for amiloride is 2-4 µM and that 10 µM amiloride was a supramaximal dose.

The effects of furosemide, an inhibitor of NKCCl, the epithelial Na⁺-K⁺-Cl⁻ cotransporter (and hence transepithelial chloride transport) was also examined in apocrine cultures. Basolateral addition of 1 mM furosemide reduced resting I^{SC} in transformed cultures by 0.8±0.30.2 µA (n=20, P<0.001).

**Table 1. The effects of amiloride and furosemide on the short circuit current in cultured apocrine (primary and proliferating) and eccrine sweat gland cells.**

| | Primary Apocrine | Proliferating Apocrine | Primary Eccrine |
|---|---|---|---|
| *Resting I^{SC}* | 8.4±0.9 | 4.5±0.8 | 8.6±1.0 |
| (µA cm⁻²) | (n=57) | (n=40) | (n=22) |
| ΔI^{SC} (µA cm⁻²) | | | |
| *Amiloride* | -6.0±0.9** | -4.2±1.0 | -7.1±0.7 |
| (10 µM) | *(n=49)* | *(n=20)* | *(n=22)* |
| Furosemide | -0.5±0.2 | -0.8±0.3 | nd |
| *(1 mM)* | (n=13) | (n=20) | |

### The role of sodium reabsorption in agonist responses

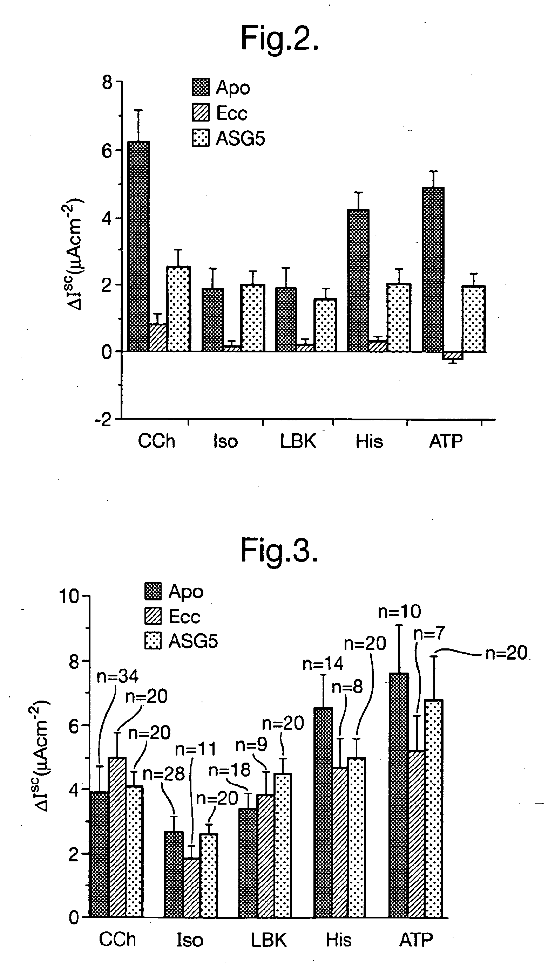

Primary apocrine and proliferating apocrine cultures were pre-treated with 10 µM amiloride to determine the role of sodium reabsorption in agonist responses. Appended Figure 2 shows Peak increases in I^{SC} in primary apocrine, eccrine and proliferating apocrine cultures in the presence of amiloride. The agonists were added to the basolateral side of cultures on Transwells. Each bar in the Figure represents a minimum of n=10. Peak increases in short circuit current in the presence of apical amiloride (10 µM) are shown. The agonists were added basolaterally: Carbachol (CCh; 20 µM), Isoprenaline (Iso; 10 pM), L-bradykinin (LBK; 170 nM), Histamine (His; 200 µM) and Adenosine triphosphate (ATP; 100 µM). Significance was tested using unpaired Student's t test and the Wilcoxon tests.
Figure 2 demonstrates that amiloride pre-treatment significantly reduced responses to CCh, His and ATP in a long term proliferating apocrine cell line, ASG5, suggesting that sodium reabsorption is a component of these responses.
Figure 3 shows peak increases in I^{SC} in primary apocrine, eccrine and proliferating apocrine cultures. Agonists were added to the basolateral side of cultures on Transwells. Peak increases in short circuit current are shown. The agonists were added basolaterally: CCh (20 µM), Iso (10 pM), LBK (170 nM), His (200 µM) and ATP (100 µM).

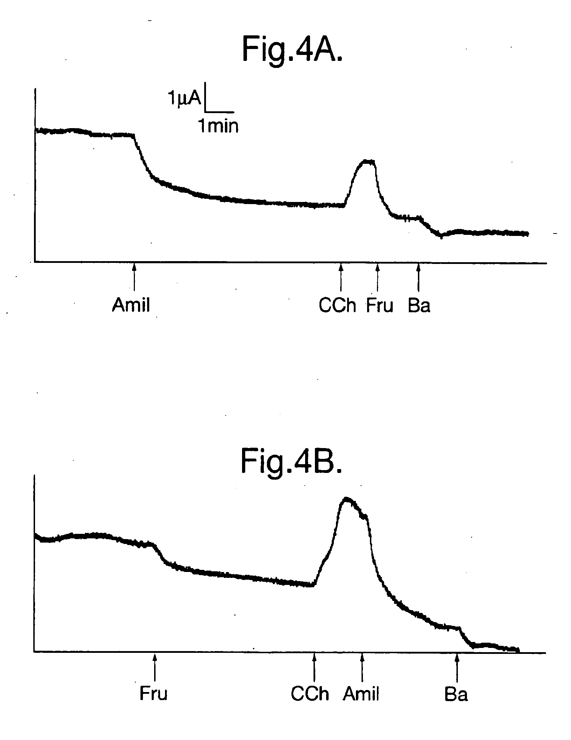

Figures 4A and 4B are representative I^{SC} records (traces) on the effects of ion channel inhibitors on the response to carbachol in ASG5 cells. Figure 4A shows an apical pre-treatment with amiloride (10 µM) and the effects of addition of 1 mM furosemide (Fru) and 50 µM barium chloride (Ba; a maxi-K channel blocker), on the CCh (20 µM) response. Figure 4B shows an apical pre-treatment with 1 mM furosemide and the effects of amiloride and barium chloride on CCh response.

The representative trace of proliferating apocrine cultures in Figure 4A also demonstrated that compared to the observed values for agonist stimulation in Figure 3, carbachol stimulation is reduced after amiloride pre-treatment.

In four cases, whole apocrine secretory coils were explanted directly onto permeable supports to determine if the primary culture and passage of apocrine cultures affected their electrophysiological properties. In the cultures examined, basal and agonist stimulated electrophysiological properties were not significantly different to primary cultures, whether in the presence or absence of amiloride (data not shown).

### The role of transepithelial chloride transport in transient agonist responses

To further characterise the ionic basis of agonist induced responses in primary and proliferating apocrine cultures, the role of transepithelial chloride transport was examined. Under chloride-free conditions, resting I^{SC} for primary and proliferating apocrine cultures was 6.0±0.8 µA cm⁻² and 3.1±0.6 µA cm⁻² respectively. Resting TEPD was - 0.9 mV for primary cultures and -1.8 mV for proliferating cultures. Addition of 10 µM amiloride reduced basal I^{SC} by 3.6 µA cm⁻² in primary cultures and by 2.5 µA cm⁻² in proliferating cultures.

Responses to carbachol, histamine and ATP were significantly reduced in both primary and proliferating apocrine cultures when compared to identical experiments in the presence of chloride (Table 2) suggesting that chloride transport does play a significant role in mediating agonist responses. Furthermore, Figure 4B shows a representative trace from proliferating cultures demonstrating that pre-treatment with furosemide reduces the magnitude of response to carbachol compared to the values in Figure 3. Addition of furosemide after agonist stimulation induced a rapid reduction in responses in both primary and transformed apocrine cultures, demonstrating the role of NKCC1 in regulating chloride uptake. (Table 3).

**Table 2. Peak agonist responses of apocrine cultures in chloride-free buffer.**

| | | | | |
|---|---|---|---|---|
| A: Primary apocrine cultures B: proliferating apocrine culture (ASG5). | | | | |

| **Agonist** | **Chloride-free** | ***n*** | **Control** | ***n*** |
|---|---|---|---|---|
| I^{SC} responses µ*A cm⁻²* | | | | |
| **A** | | | | |
| Carbachol (20 µM) | 0.7 ± 0.6 * | 5 | 3.9 ± 0.7 | 34 |
| Histamine (200 µM) | 1.7 ± 0.5 ** | 5 | 6.4 ± 1.3 | 14 |
| ATP (100 µM) | 2.9 ± 0.9 * | 9 | 7.4 ± 1.8 | 10 |
| **B** | | | | |
| Carbachol (20 µM) | 3.0 ± 0.8 * | 6 | 4.0 ± 0.7 | 20 |
| Histamine (200 µM) | 3.3 ± 0.8 ** | 6 | 4.8 ± 1.3 | 20 |
| ATP (100 µM) | 4.0 ± 0.5* | 6 | 6.3 ± 1.8 | 20 |

**Table 3. Furosemide inhibition of agonist responses in primary and long term proliferating (AGS5) apocrine cultures.**

| **Agonist** | **Inhibition by furosemide (90 µM)** | |
|---|---|---|
| | Δ **I^{SC}** ,µ*A cm⁻²* | |
| | **Primary** | **Proliferating** |
| Carbachol (≥ 20 µM) | -2.0 ± 0.5 | -1.1±0.4 |
| | (*n=4*) | (*n=10*) |
| Isoprenaline (10 µM) | 0.8 ± 0.2 | -1.2±0.3 |
| | (*n=3*) | *(n=10)* |
| Histamine (200 µM) | -1.3 ± 0.2 | -1.0±0.4 |
| | *(n=6)* | *(n=10)* |
| ATP (100 µM) | -2.6 ± 0.5 | -1.2±0.3 |
| | *(n=8)* | *(n=10)* |

Agonist responses were significantly reduced with amiloride pre-treatment when compared to amiloride pre-treatment in chloride containing buffer, but they were not entirely abolished, particularly in the proliferating cultures, suggesting that other ion transport pathways may also play a role in these responses (Table 4).

**Table 4. Peak agonist responses of apocrine cultures in chloride-free buffer after amiloride pre-treatment.**

| | | | | |
|---|---|---|---|---|
| A: Primary apocrine cultures B: proliferating apocrine culture. | | | | |

| **Agonist** | **Chloride**-**free** | ***n*** | **Control** | ***N*** |
|---|---|---|---|---|
| **I**^{SC} **responses** µ*A cm⁻²* | | | | |
| **A** | | | | |
| Carbachol (200 µM) | 1.7 ± 1.2 ** | 9 | 6.3 ± 1.4 | 11 |
| Isoprenaline (≥ 1 µM) | 0.7 ± 0.3 * | 15 | 1.8 ± 0.4 | 20 |
| LBK (170 nM) | 0.6 ± 0.4 * | 11 | 2.0 ± 0.5 | 18 |
| Histamine (200 µM) | 2.4 ± 1.4 | 8 | 4.2 ± 0.5 | 19 |
| ATP (100 µM) | 4.3 ± 2.4 | 6 | 4.9 ± 0.7 | 24 |
| B | | | | |
| Carbachol (200 µM) | 1.3±0.3 | 6 | 2.4±0.6 | 20 |
| Isoprenaline (≥ 1 µM) | 0.8±0.2 | 6 | 1.8±0.4 | 20 |
| LBK (170 nM) | 0.9±0.3 | 6 | 1.6±0.3 | 20 |
| Histamine (200 µM | 1.0±0.2 | 6 | 1.9±0.4 | 20 |
| ATP (100 µM) | 0.8±0.2 | 6 | 1.8±0.3 | 20 |

Taken together these results indicate that the proliferating cell line continued to exhibit the same characteristics as the primary cultures.

### Steroidogenesis in a long term proliferating apocrine gland cell line

Human skin and its associated appendages are capable of a variety of metabolic functions, including those involved in the metabolism and synthesis of hormones. Androgens (sex steroid hormones), are synthesized from the parental precursor cholesterol in a biosynthetic pathway requiring numerous enzymatic conversions. As apocrine glands become functional during puberty, it is likely that androgens play some role in modulating gland activity. This is supported further by the fact that the androgen receptor has been detected in the secretory cells of apocrine glands residing in axillary skin (Beier et al, (2005) Histochem. Cell Biol., 123: 61-65). Since the local or intracrine formation of steroids potentially plays an important role in mediating cell function (Labrie, F. et al, (1991), Mol. Cell Endocrinol., 78: 113-118), the expression of transcripts responsible was investigated for the formation of active androgens and estrogens in the indefinitely proliferating apocrine cell line herein, as well as their associated receptors.

### Materials & Methods in the investigation Chemicals

Taq DNA polymerase and molecular marker III were purchased from Roche, Mannheim Germany. Taq DNA polymerase was used according to the suppliers' recommendations. All other reagents were obtained from Sigma-Aldrich, Gillingham, UK.

### Strains, plasmids, media and culture conditions

*E. coli* strain TOP10 (Invitrogen, Carlsbad, CA, USA) was used as the host strain in all cloning procedures. Predigested vector pCR^{®}4-TOPO^{®} containing poly-T overhangs was obtained from Invitrogen and used according to the suppliers instructions. Bacteria were cultivated in LB medium (10 g/l tryptone, 5 g/l yeast extract, 5 g/l NaCl) supplemented with the appropriate selection pressure (kanamycin 50 µg/ml) on a rotary shaker (250 rpm) at 37°C.

### Cell material

Long term proliferating apocrine gland cells indicative of indefinite proliferation, were grown as described hereinbefore. For cloning purposes cells were harvested after 10 days growth at 37°C.

### Oligonucleotide synthesis and sequencing

All oligonucleotides used were synthesized by Sigma-Genosys Ltd, Pampisford, UK. Sequencing was performed using an ABI Prism 3100 Genetic Analyser using the universal M13 forward and reverse primers.

### Isolation of plasmid DNA

The Qiagen (Crawley, UK) mini-prep kit was used to obtain plasmid DNA for cloning and sequencing.

### Cloning

Fragments of transcripts involved in steroid androgen metabolism were cloned from cDNA derived from indefinitely proliferating apocrine gland cells. RNA was isolated from the cells using the RNAqueous™-4PCR kit from Ambion, Huntingdon, UK. First strand cDNA synthesis was performed using the Ambion RETROscript™ kit using oligo(dt). The PCR reaction programme used for amplification was: 1 cycle (120 s @ 95 °C), 30 cycles (30 s @ 95 °C, 30 s @ 60 °C, 45 s or 60 s @ 72 °C), 1 cycle (7 min @ 72 °C) with Taq DNA polymerase). The primers used to clone fragments from each transcript are outlined in Table 5.

**Table 5. Primers used to clone gene fragments involved in androgen metabolism.**

| Primer sequence | Gene | Expected size (bp) |
|---|---|---|
| Forward 5'- | Steroid | 392 |
| CTTCACCCCCAACTTCAACCCCG-3' | sulphatase | |
| Reverse 5'- CCA CAT GCG TCT GTC | | |
| TGG TCC CC-3' | | |
| Forward 5'- | **3**β-hydroxy-Δ-**5**- | 813 |
| GCCAGTCTTCATCTACACCAGTAG C-3' | steroid | |
| Reverse 5'- | dehydrogenase | |
| CTCTGTCATCCTTAAATCACTGAGTC-3' | 1 | |
| Forward 5'- | Hydroxysteroid | 436 |
| CCTCATCCATTGTAACATCACCTCC-3' | **17**β- | |
| Reverse 5'- | dehydrogenase | |
| CTCACCGCCTGGCTACCTGACC-3' | 3 | |
| Forward 5'- | Hydroxysteroid | 990 |
| GACAAGTGACAGGGAATGGATTCC-3' | **17**β- | |
| Reverse 5'- | dehydrogenase | |
| CAGGGCTTCTGGTAGACATCAGG-3' | 5 | |
| Forward 5'- | Hydroxysteroid | 722 |
| GAAGTGTGAGTGCGCGAAGATGCG-3' | **17**β- | |
| Reverse 5'- | dehydrogenase | |
| GTGCTGGAATTATAGGCATGAGCCAC-3' | 7 | |
| Forward 5'- | 5α Reductase | 773 |
| CCAGCCCTGGCGATGGCAACG-3' | | |
| Reverse 5'- | | |
| GAAATTCTGACCTGTTACACAGTAGG-3' | | |
| Forward 5'- | Aromatase | **447** |
| GCCTGTCGTGGACTTGGTCATGCG-3' | | |
| Reverse 5'- | | |
| GAGTAGGTACTGACCAGCCTTCTC-3' | | |
| Forward 5'- | Estrogen | 564 |
| CTTGGAGAGCTGTTGGATGGAGGTG-3' | receptor β | |
| Reverse 5'-CAGGGCCAGGCGTCACTGAGA | | |
| C-3' | | |
| Forward 5'- | Androgen | 564 |
| CTGGATGGGGCTCATGGTGTTTG-3' | receptor | |
| Reverse 5'- | | |
| GTTTCCAATGCTTCACTGGGTGTGG-3' | | |

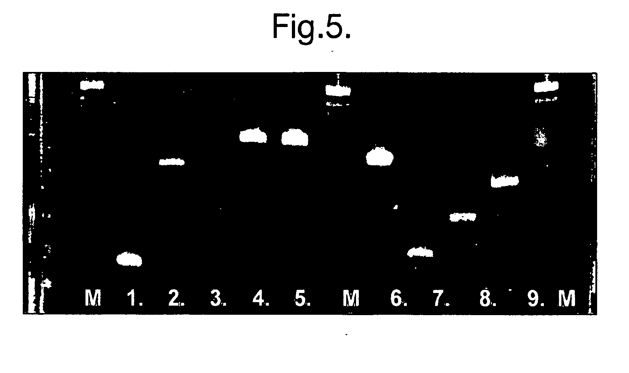

All the gene fragments that primer pairs were designed for, as outlined in table 5, could be detected. Appended Figure 5 displays a 1.2% agarose gel of all the PCR products produced.

Figure 5 shows the expression of gene fragments :
1. Steroid sulphatase; 2. 3βhydroxy-Δ-5-steroid dehydrogenase 1; 3. Hydroxysteroid 17β-dehydrogenase 3; 4. Hydroxysteroid 17β-dehydrogenase 5; 5. Hydroxysteroid 17β-dehydrogenase 7; 6. 5α Reductase I; 7. CYP19A1 Aromatase; 8. Androgen receptor; 9. Estrogen receptor β; M. DNA Marker III.

These PCR products were then cloned into the TOPO vector ready for sequencing. Sequencing was performed on each PCR product to confirm the identity of each cloned fragment. The results of the gene sequencing are given below.
Steroid sulphatase fragment sequence 
TCCCTTCTTCACCCCCAACTTCAACCCCGTGGGTTCCAACGGATGCTTTGCCACACACG
TGTGCTTCTGTTTCGGGAGTTATGTCACCCATCACGACCCACCTTTACTCTTTGATATT
TCCAAAGATCCCAGAGAGAGAAACCCACTAACTCCAGCATCCGAGCCCCGGTTTTATGA
AATCCTCAAAGTCATGCAGGAAGCTGCGGACAGACACACCCAGACCCTGCCAGAGGTGC
CCGATCAGTTTTCATGGAACAACTTTCTTTGGAAGCCCTGGCTTCAGCTGTGCTGTCCT
TCCACCGGCCTGTCTTGCCAGTGTGATAGAGAAAAACAGGATAAGAGACTGAGCCGCTA
GCAGCGCCTGGGGACCAGACAGACGCATGTGGAAGGGC
3β-hydroxy-Δ-5-steroid dehydrogenase 1 fragment sequence 
GCCCTTCTCTGTCATCCTTAAATCACTGAGTCTTGGACTTCACGGTCTCCTTGTGCCAG
TCCACAAGGGAAACCCACTCCACGGTTCTAACAGACTCAGGTACATTCTTCTTGGCTTC
CTCCCAGCTGTAAAGTGGCTTATATGCCAGATCTTGCTGAGCCTTCTTGTAAGAGAAGG
TGAACACGCTATTTGACAATGTCACTCTGTGGTGGTTGAAGGGTGTCGATAGGTGTAAA
TTGGCCTCAGCAGGAAGCTCACTATTTCCAGCAGGAAGCCAATCCAGTACATCAGAGAT
AAAGGAAGGCTCCATCTAGAATCAAGGCAGAGGCCGAACTCTTTGCTCAGGATGTAATT
AAGGTTATCATAGCTTTGGTGAGGCATGTCATCTGAGATGTAGTAGAACTGTCCTCGGA
CACTTGGGGCCTTCTTGGGCTCCCGCAGGGCCTCAAGGCCAGAATGTGGGCCCAGGCCA
 
TGTTTCCAATATAGACTGGGTTGACTCTGGAGAACTTGCTGAAACTTGACAGGATCCCA
TGGTTGTTTAGGGCCTCATTTATACCGGCAGAAAGGATTGGGCTTCCTTCCCCATAGAT
AAACATTGGTCTTAAGGCACAAGTGTACAAGGTGCCACCGTTTTTCAGAGTCCACCCAT
TAGCCGCCAGCACAGCCTTCTCAGCAAGCTTTTTGCTGTGTGGGTATGGAGCGTACCAT
GTGTTTTCCAGAGGCTCTTCTTCGTGACTGTTCTGGATGATTTCCTTGTAGGAATTGGG
CCCGGCTACCTCTGGGCTACTGGTGTAGATGAAGACTGGCAAGGGC
Hydroxysteroid 17β-dehydrogenase 3 fragment sequence 
GCCCTTCTCACCCNCTGGCTACCTGACCTTGGTGTTGAGCTTCAGGTATGCCACATAGT
GTGTCAGGAGCAGCCTTTGGAAGGCACCGCTGTAGAAGGCCCAGGCCGGATCAGGCTC
AGAAAGCCCGCCAAGATTTCATGGGCAAGGCAGCCGCAGGTTTCACCTCCAATTGTGAC
ATAATTCAATGACTCTTTGACAAACTCATCAGCAGTCTTGGTTATCACATTTGTATTTA
GATACTTTGTCATTGCAGTCGGGACAGCATATGGGGTCAGCACCTGGATGATGACTTCT
TTTGCTTTATATTCCTCTTGCAGGGCCTTGGAAAATGCGCACACAAACGCCTTGGAAGC
TGAGTACATGGAGTAGAGAGGCCAAGGAAACAGGGCTATCCCAGAAGAAATGTTCAGGA
TGAGACCTTTCTGCCTTGATTCCATATGTTTCAAAATTAGCTGTGTCATCTTGACTACG
GAGGTGATGTTACAATGGATGAGGAAGGGC
Hydroxysteroid 17β-dehydrogenase 5 fragment sequence 
GACAAGTGACAGGGAATGGATTCCAAACACCAGTGTGTAAAGCTAAAGGATGGCCACTT
CATGCCTGTATTGGGATTTGGCACCTATGCACCTCCAGAGGTTCCAAGAAGTAAAGCTT
TGGAGGTCACAAAATTAGCAATAGAAGCTGGGTTCCGCCATATAGATTCTGCTCATTTA
TACAATAATGAGGAGCAGGTTGGACTGGCCATCCGAAGCAAGATTGCAGATGGCAGTGT
GAAGGGAGAAGACATATTCTACACTTCAAAGCTTTGGTCCACTTTTCATCGACCAGAGT
TGGTCCGACCAGCCTTGGAAAACTCACTGAAAAAAGCTCAATTGGACTATGTTGACCTC
TATCTTATTCATTCTCCAATGTCTCTAAAGCCAGGTGAGGAACTTTCACCAACAGATGA
AAATGGAAAAGTAATATTTGACATAGTGGATCTCTGTACCACCTGGGAGGCCATGGAGA
AGTGTAAGGATGCAGGATTGGCCAAGTCCATTGGGGTGTCAAACTTCAACCGCAGGCAG
CTGGAGATGATCCTCAACAAGCCAGGACTCAAGTACAAGCCTGTCTGCAACCAGGTAGA
ATGTCATCCGTATTTCAACCGGAGTAAATTGCTAGATTTCTGCAAGTCGAAAGATATTG
TTCTGGTTGCCTATAGTGCTCTGGGATCTCAACGAGACAAACGATGGGTGGACCCGAAC
 
TCCCCGGTGCTCTTGGAGGACCCAGTCCTTTGTGCCTTGGCAAAAAGCACAAGCGAAC
CCCAGCCCTGATTGCCCTGCGCTACCAGCTGCAGCGTGGGTTGTGGTCCTGGCCAAGA
GCTACAATGAGCAGCGCATCAGACAGAACGTGCAGGTTTTTGAGTTCCAGTTGACTGCA
GAGGACATGAAAGCCATAGATGGCCTAGACAGAAATCTCCACTATTTTAACAGTGATAG
TTTTGCTAGCCACCCTAATTATCCATATTCAGATGAATATTAACATGGAGAGCTTTGCC
TGATGTCTACCAGAAGCCCTG
Hydroxysteroid 17β-dehydrogenase 7 fragment sequence 
GCCCTTGAAGTGTTAGTGCGCGAAGATGCGAAAGGTGGTTTTGATCACCGGGGCTAGCA
GTGGCATTGGCCTGGCCCTCTGCAAGCGGCTGCTGGCGGAAGATGATGAGCTTCATCTG
TGTTTGGCGTGCAGGAACATGAGCAAGGCAGAAGCTGTCTGTGCTGCTCTGCTGGCCTC
TCACCCCACTGCTGAGGTCACCATTGTCCAGGTGGATGTCAGCAACCTGCAGTCGGTCT
TCCGGGCCTCCAAGGAACTTAAGCAAAGGTTTCAGAGATTAGACTGTATATATCTAAAT
GCTGGGATCATGCCTAATCCACAACTAAATATCAAAGCACTTTTCTTTGGCCTCTTTTC
AAGAAAAGTGATTCATATGTTCTCCACAGCTGAAGGCCTGCTGACCCAGGGTGATAAGA
TCACTGCTGATGGACTTCAGGAGGTGTTTGAGACCAATGTCTTTGGCCATTTTATCCTG
ATTCGGGAACTGGAGCCTCTCCTCTGTCACAGTGACAATCCATCTCAGCTCATCTGGAC
ATCATCTCGCAGTGCAAGGAAATCTAATTTCAGCCTCGAGGACTTCCAGCACAGCAAAG
GCAAGGAACCCTACAGCTCTTCCAAATATGCCACTGACCTTTTGAGTGTGGCTTTGAAC
AGGAACTTCAACCAGCAGGGTCTCTATTCCAATGTGGCCTGTCCAGGTACAGCATTGAC
CAATTTGACATATGGAATTCTGCCTCCGTTTATATGGACGCTGTTGATGCCGGCAATAT
TGCTACTTCGCTTTTTTGCAAATGCATTCACTTTGACACCATATAATGGAACAGAAGCT
CTGGTATGGCTTTTCCACCAAAAGCCTGAATCTCTCAATCCTCTGATCAAATATCTGAG
TGCCACCACTGGCTTTGGAAGAAATTACATTATGACCCAGAAGATGGACCTAGATGAAG
ACACTGCTGAAAAATTTTATCAAAAGTTACTGGAACTGGAAAAGCACATTAGGGTCACT
ATTCAAAAAACAGATAATCAGGCCAGGCTCAGTGGCTCATGCCTATAATTCCAGCAC
5α Reductase I fragment sequence 
GCCCTTCCAGCCCTGGCGATGGCAACGGCGACGGGGTGGCGGAGGAGCGCCTGCTGGC
CGCGCTCGCCTACCTGCAGTGCGCCGTGGGCTGCGCGGTCTTCGCGCGCAATCGTCAGA
CGAACTCAGTGTACGGCCGCCCCGCGTCTCCGCAGCGCGCCCAACTGCATCCTCCTGGC
 
CATGTTCCTCGTCCACTACGGGCATCGGTGCTTAATTTACCCATTTCTGATGCGAGGAG
GAAAGCCTATGCCACTGTTGGCGTGTACAATGGCGATTATGTTCTGTACCTGTAACGGC
TATTTGCAAAGCAGATACTTGAGCCATTGTGCAGTGTATGCTGATGACTGGGTAACAGA
TCCCCGTTTTCTAATAGGTTTTGGCTTGTGGTTAACGGGCATGTTGATAAACATCCATT
CAGATCATATCCTAAGGAATCTCAGAAAACCAGGAGATACTGGATACAAAATACCAAGG
GGAGGCTTATTTGAATACGTAACTGCAGCCAACTATTTTGGAGAAATCATGGAGTGGTG
TGGCTATGCCCTGGCCAGCTGGTCTGTCCAAGGCGCGGCTTTTGCTTTCTTCACGTTTT
GTTTTTTATCTGGTAGAGCAAAAGAGCATCATGAGTGGTACCTCCGGAAATTTGAAGAG
TATCCAAAGTTCAGAAAAATTATAATTCCATTTTTGTTTTAAGTGCGTTTTTCATGAAA
TTATCTTCAACTTGAAGCTTTCCAATGGCGCTTCTCTATGGACTTTGTAAATAAGTTAT
ATCTTTGTAATTTTCCTGCTACTTTATCATTTTCAAGATGTCCTCTAGGAATTTTTTTT
CTAGTAATTTTGCAATCTACCTAATAAGTACCTAAATACGCTGAAATGGAGGTTGAATA
TCCTACTGTGTAACAGGTCAGAATTNCANGGGC
 CYP19A1 Aromatase fragment sequence

GCCCTTGAGTAGNTACTGACCAGCCTTCTCTAGTGTTCCAGACACCTGTCTGAGTTTCT
TGGGGTAAAGATCATTTCCAGCATGTTTTTAGTCTCATCTGGGTGCAAGGACAAGTCGT
GTATCTTCTGTATGCTCTCAACACACTGTCCTTGCAATGTCTTCACGTGGAATCGTCTC
AGAAGTGTAACGAGGATGGCTTTCATCATCACCATGGCGATGTACTTTCCTGCACAGCC
ACGGGGCCCAAAGCCAAATGGCTGAAAGTACCTATAAGGAACATTCTTTGCAAAATTTT
CAAGAGTAAATTCATTGGGTTTGGGGAAAAACTCGAGTCTGTGCATCCTTCCAATATTC
AGGATAATGTTTGTCCCCTTTTTCACTGGGTAGCCATCGATTACATCATCTTCTAAGGC
TTTGCGCATGACCAAGTCCACGACAGGCAAGGGC

Androgen receptor fragment sequence 
GCCCTTGTTTCCNTGCTTCACTGGGTGTGGAAATAGATGGGCTTGACTTTCCCAGAAAG
GATCTTGGGCACTTGCACAGAGATGATCTCTGCCATCATTTCCGGAAAGTCCACGCTCA
CCATGTGTGACTTGATTAGCAGGTCAAAAGTGAACTGATGCAGCTCTCTCGCAATAGGC
TGCACGGAGTCCAGGAGCTTGGTGAGCTGGTAGAAGCGTCTTGAGCAGGATGTGGGATT
TTTTCTTTTGCATGCAATGATACGATCGAGTTCCTTGATGTAGTTCATTCGAAGTTCAT
CAAAGAATTTTTGATTTTTCAGCCCATCCACTGGAATAATGCTGAAGAGTAGCAGTGCT
 
TTCATGCACAGGAATTCCTGGGGGGTGATTTGGAGCCATCCAAACTCTTGAGAGAGGTG
CCTCATTCGGACACACTGGCTGTACATCCGGGACTTGTGCATGCGGTACTCATTGAAAA
CCAGATCAGGGGCGAAGTAGAGCATCCTGGAGTTGACATTGGTGAAGGATCGCCAGCCC
ATGGCAAACACCATGAGCCCCATCCAGAAGGGC
Estrogen receptor β fragment sequence

GCCCTTCAGGGNCNNGGCGTCACTGAGACTGTGGGTTCTGGGAGCCCTCTTTGCTTTTA
CTGTCCTCTGCCGGGCTGCACTCGGACCCCGTGATGGAGGACTTGCACCCGCGAAGCAC
GTGGGCATTCAGCATCTCCAGCAGCAGGTCATACACTGGGACCACATTTTTGCACTTCA
TGTTGAGCAGATGTTCCATGCCCTTGTTACTCGCATGCCTGACGTGGGACAGGAGCATC
AGGAGGTTAGCCAGGCGCATGGATTGCTGCTGGGAGGAGATGCCGCTCTTGGCAATCAC
CCAAACCAAAGCATCGGTCACGGCGTTCAGCAAGTGAGCCAGCTTCCGGCTGCTGTCAG
CATCCTGGGTCGCTGTGACCAGAGGGTACATACTGGAATTGAGCAGGATCATGGCCTTG
ACACAGAGATATTCTTTGTGTTGGAGTTTTAACTCTCGAAACCTTGAAGTAGTTGCCAG
GAGCATGTCAAAGATTTCCAGAATTCCTTCTACGCATTTCCCCTCATCCGTCTGTAATC
CCAACAATTTGGGAGGCTGAGGCTGGGGATCACTTGAAGTCAGGACCTCGAGACCAGC
TTGGCCAACATGGTAAAACCCCGTCTCCACTGTCCAGAACAAGATCTGGAGCAAAGATG
AGCCTGCCGGGGTGGTCAATTGAGCGCCACATCAGCCCCATCATTAACACCTCCATCCA
ACAGCTCTCCAAGAAGGGC

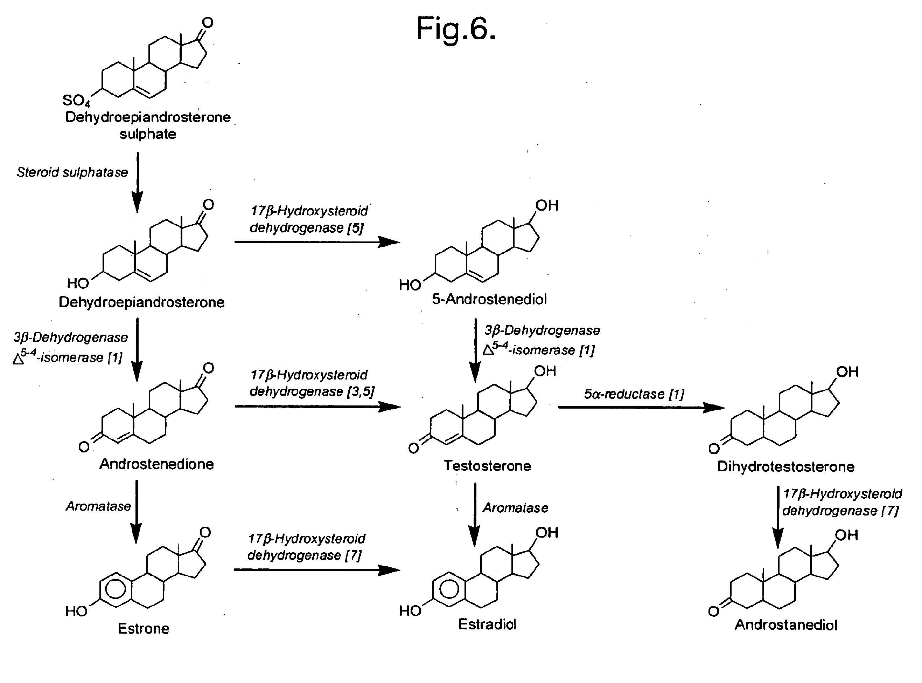

These results clearly demonstrate that the indefinitely proliferating apocrine gland cell line is expressing numerous genes required for androgen and estrogen synthesis. Appended Figure 6 outlines the steroid synthesis pathway that can be elucidated given the genes that have shown to be expressed in the indefinitely proliferating apocrine gland cell line. In addition the cells also express the necessary receptors required to mediate the intracellular response to the production of these androgens and estrogens i.e. the androgen and estrogen β receptor. Interestingly, no transcript for the expression of the estrogen α receptor could be detected (data not shown). This is in agreement with a previous study that demonstrated that the estrogen β receptor could be detected in apocrine gland secretory cells in axillary skin sections but not the estrogen α receptor, using immunohistochemical staining for both receptor types (Beier, K. et al (2005) Histochem. Cell Biol., 123: 61-65). The same study also demonstrated the presence of the androgen receptor in apocrine gland secretory cells.

The data in appended Figure 6 demonstrates that the indefinitely proliferating apocrine cell line retains many of the steroidogenic features of secretory apocrine gland cells observed *in-vivo* and primary culture. This cell line provides an excellent tool for studying steroid metabolism in apocrine glands and the role of steroids in regulating gland activity. Additionally, the cell line will serve as an invaluable tool for evaluating the efficacy of compounds in regulating gland activity via interference of the steroidogenic pathways in these cells.

### Cell markers of apocrine secretory activity in the indefinitely proliferating apocrine gland cell line.

The major function of the apocrine sweat gland is to produce a lipid rich secretion which is delivered to the skin surface via the canal of the hair follicle in direct response to emotional stimuli. The secretion is non-odorous, but undergoes bacterial decomposition which results in the generation of axillary malodours. The secretory function of the apocrine glands is associated with a number of proteins involved in these key processes. The *ABCC11* protein belongs to the family of ATP-binding cassette transporters involved in the efflux of purine and pyrimidine nucleotide analogs such as cAMP and cGMP (Guo et al., (2003) J. Biol. Chem., 278: 29509-29514). This protein has been implicated in the secretion of earwax in the ceruminous apocrine glands of humans (Yoshiura et al., (2006) Nat. Gen., 38: 324-330). Human earwax normally consists of dry and wet types. Dry earwax is frequent in East Asians, whereas wet earwax is common in other populations. A SNP, 538G→A in the *ABCC11* gene is considered to be responsible for determination of earwax type. Cells with the A allele show a lower excretory activity for cGMP than those with the G allele. The A allele frequency shows a global North-South and East-West downward geographical gradient. Worldwide, it is highest in Chinese and Koreans where a dry earwax-type is retained amongst the various ethnic populations in these regions. Increased levels of axillary odour are associated with wet-type earwax, which is considered to be a direct consequence of axillary apocrine gland function.

Another protein which has been shown to play an important role in apocrine gland secretion is apolipoprotein D by Spielman, A.I.(1995) Experientia, 50: 40-47. This protein has been shown to act as a carrier vehicle for the abundant odour molecule E-3-methyl-2-hexanoic acid (3M2H). Studies have demonstrated that in apocrine secretions 3M2H is carried to the skin surface bound by two proteins, apocrine secretion odour-binding proteins 1 and 2 (ASOB1 and ASOB2). The ASOB2 protein was subsequently identified as apolipoprotein D (apoD), a known member of the α_{2µ}-microglobulin superfamily of carrier proteins also known as lipocalins (Zeng et al., (1996), **93:** 6626-6630). Immunoreactivity for apoD has been localised to the apocrine glands in axillary tissue sections (Spielman et al., (1998) **134,** 813-818) indicating that at least one of the glycoprotein carriers for 3M2H is localized in the apocrine glands.

Gross cystic disease fluid is a pathologic secretion from breast composed of several glycoproteins, including GCDFP-15. GCDFP-15 has been localized in the apocrine metaplastic epithelium lining breast cysts and in apocrine glands of the axilla, vulva, eyelid, and ear canal (Mazoujian et al., 1983 Am. J. Pathol., 110: 105-112). GCDFP-15 is identical to the Gp17/ secretory actin binding protein (SABP/extra-parotid glycoprotein (EP-GP)-which has been identified in the seminal vesicles, the salivary glands, and the sweat glands (Autiero et al., (1991); Exp. Cell Res., 197: 268-271), this protein is also identical to the prolactin inducible protein (PIP) (Murphy et al., (1987); J. Biol. Chem., 262: 15236-15241). GCDFP-15 is therefore a specific tissue marker of apocrine epithelium.

Zinc-α-glycoprotein (ZAG) is a 43 kDa soluble glycoprotein first isolated from human plasma (Burgi et al., (1961); J. Biol Chem; 236, 1066-1074) and subsequently found in secretory epithelia cells of liver, breast, the gastrointestinal tract, and sweat glands (Tada et al., (1991) J. Histochem Cytochem, 39, 1221-1226). ZAG is over-expressed in certain malignant tumours such that it may serve as a cancer maker Diez-Itza et al., (1993), Eur. J. Cancer A 29, 1256-1260; Hale et al., (2001), Cancer Res., 7, 846-853). The biological functions of ZAG are largely unknown, but it has been shown to act as a lipid mobilizing factor (Todorov et al., (1998), Cancer Res., 58, 2353-2358) and a novel adipokine, which may be involved in the local regulation of adipose tissue function (Bao, et al., (2005) FEBS Letters, 579, 41-47).

As ABCC11, ApoD, GCDFP-15 and Zinc-α-glycoprotein play important roles in the secretion and transport processes of apocrine glands, we investigated the expression of transcripts responsible for the translation of these proteins in the indefinitely proliferating apocrine cell line.

The primers used to clone the gene transcript fragments are displayed in Table 6.

**Table 6. Primers used to clone gene fragments involved in apocrine secretory processes.**

| Primer sequence | Gene | Expected size (bp) |
|---|---|---|
| Forward 5'- | ABCC11 | 254 |
| CACCATCCCTCCACTGTCAGTCC-3' | | |
| Reverse 5'- | | |
| CAACATTCCCCAACTGCTCTTCTG-3' | | |
| Forward 5'- | Apolipoprotein D | 583 |
| CGGTGCGGCAGAGGGACAAG-3' | | |
| Reverse 5'- | | |
| GGGGAAAGCGAAGCAGAAGTAAC-3' | | |
| Forward 5'- | GCDFP-15 | 340 |
| CCAGCCCTGCCACCCTGCTCC-3' | | |
| Reverse 5'- | | |
| GCAGATGCCTAATTCCCGAATAAC-3' | | |
| Forward 5' - | Zinc-α- | 518 |
| GGTGGAAGGAATGGAGGATTGG-3' | glycoprotein | |
| Reverse 5'- | | |
| GTGGCAGGAGTAGGGGGCTG-3' | | |

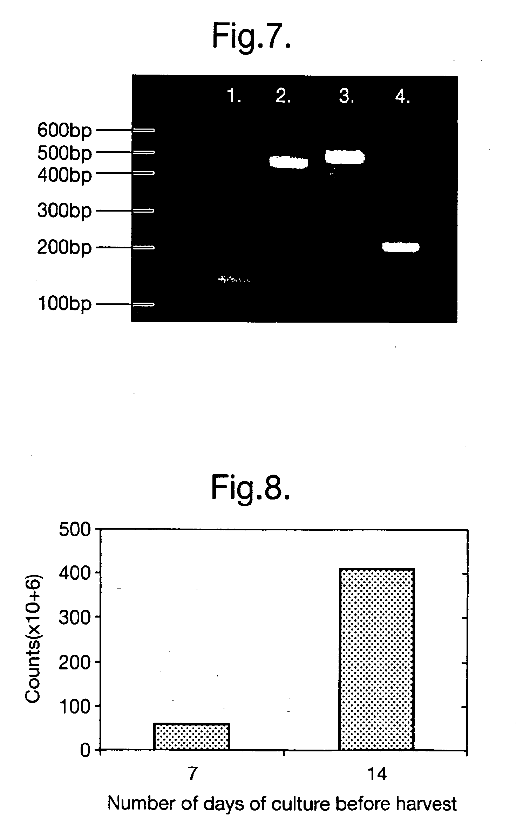

All the gene fragments that primer pairs were designed for, as outlined in table 6, could be detected. Appended Figure 7 displays a 1.2% agarose gel of all the PCR products produced.

Appended Figure 7. Agarose gel displaying the apocrine marker discloses: The following products are displayed in lanes 1 to 4 respectively.
1. ABCC11 transporter;
2. Apolipoprotein D;
3. Zinc-α-glycoprotein;
4. GCDFP-15

These PCR products were then cloned into the TOPO vector ready for sequencing. Sequencing was performed on each PCR product to confirm the identity of each cloned fragment. The results of the gene sequencing are given below.
ABCC11 fragment sequence 
CACCATCCCTCCACTGTCAGTCCATGATGCCTCAGACAAAAATGTCCAAAGGCTTCACC
GTCTTTGGGAAGAAGTCTCAAGGCGAGGGATTGAAAAGCTTCAGTGCTTCTGGTG
ATGCTGAGGTTCCAGAGAACAAGGTTGATTTCGATGCACTTCTGGGCATCTGCTTCTG
CATTGCCAGTGTACTCGGGCCAATATTGATTATACCAAAGATCCTGGAATATTCAGAAG
AGCAGTTGGGGAATGTTG
Apolipoprotein D fragment sequence 
CGGTGCGGCAGAGGGACAAGCATTTCATCTTGGGAAGTGCCCCAATCCTCCGGTGCAGG
AGAATTTTGACGTGAATAAGTATCTCGGAAGATGGTACGAAATTGAGAAGATCCCAACA
ACCTTTGAGAATGGACGCTGCATCCAGGCCAACTACTCACTAATGGAAAACGGAAAGAT
CAAAGTGTTAAACCAGGAGTTGAGAGCTGATGGAACTGTGAATCAAATCGAAGGTGAAG
CCACCCCAGTTAACCTCACAGAGCCTGCCAAGCTGGAAGTTAAGTTTTCCTGGTTTATG
CCATCGGCACCGTACTGGATCCTGGCCACCGACTATGAGAACTATGCCCTCGTGTATTC
CTGTACCTGCATCATCCAACTTTTTCACGTGGATTTTGCTTGGATCTTGGCAAGAAACC
CTAATCTCCCTCCAGAAACAGTGGACTCTCTAAAAAATATCCTGACTTCTAATAACATT
GATGTCAAGAAAATGACGGTCACAGACCAGGTGAACTGCCCCAAGCTCTCGTAACCAGG
TTCTACAGGGAGGCTGCACCCACTCCATGTTACTTCTGCTTCGCTTTCCCC
GCDFP-15 fragment sequence 
GCCCTTCCAGCCCTGCCACCCTGCTCCTGGTTCTCTGCCTGCAGTTGGGGGCCAACAAA
GCTCAGGACAACACTCGGAAGATCATAATAAAGAATTTTGACATTCCCAAGTCAGTACG
TCCAAATGACGAAGTCACTGCAGTGCTTGCAGTTCAAACAGAATTGAAAGAATGCATGG
TGGTTAAAACTTACCTCATTAGCAGCATCCCTCTACAAGGTGCATTTAACTATAAGTAT
 
ACTGCCTGCCTATGTGACGACAATCCAAAAACCTTCTACTGGGACTTTTACACCAACAG
AACTGTGCAAATTGCAGCCGTCGTTGATGTTATTCGGGAATTAGGCATCTGCAAGGGC
Zinc-α-glycoprotein

GCCCTTGGTGGAAGGAATGGAGGATTGGAAGCAGGACAGCCAACTTCAGAAGGCCAGGG
NAGGACATCTTTATGGAGACCCTGAAAGACATTGTGGAGTATTACAACGACAGTAACGG
GTCTCACGTATTGCAGGGAAGGTTTGGTTGTGAGATCGAGAATAACAGAAGCAGCGGAG
CATTCTGGAAATATTACTATGATGGAAAGGACTACATTGAATTCAACAAAGAAATCCCA
GCCTGGGTCCCCTTCGACCCAGCAGCCCAGATAACCAAGCAGAAGTGGGAGGCAGAACC
AGTCTATGTGCAGCGGGCCAAGGCTTACCTGGAGGAGGAGTGCCCTGCGACTCTGCGGA
AATACCTGAAATACAGCAAAAATATCCTGGACCGGCAAGATCCTCCCTCTGTGGTGGTC
ACCAGCCACCAGGCCCCAGGAGAAAAGAAGAAACTGAAGTGCCTGGCCTACGACTTCTA
CCCAGGGAAAATTGGTGTGCACTGGACTCGGGCCGGCGAGGTGCAGGAGCCTGAGTTAC
GGGGAGATGTTCTTCACAATGGAAATGGCACTTACCAGTCCTGGGTGGTGGTGGCAGTG
CCCCCGCAGGACACAGCCCCCTACTCCTGCCACAAGGGC

The indefinitely proliferating apocrine cell line expresses the *ABCC11* gene, which is associated with cellular secretory processes. The sequencing data confirmed the identity of the ABCC11 transporter gene. The genotype of the gene in this cell line is G (538G→A highlighted and underlined) and is associated with wet-earwax type and increased levels of axillary odour. The apolipoprotein D carrier gene is also expressed, indicating that the necessary machinery for the transport of odorants is also being manufactured in the cell line. The data provides further evidence of the apocrine phenotype of this cell line and its usefulness in providing a robust tool for the investigation of apocrine gland biology.

### Cell chemical composition - Materials and Methods

Indefinitely proliferating apocrine cells (passage 44) were harvested at 7 and 14 days, respectively. The cells were centrifuged at 1000 rpm for 5 mins washed once in PBS recentrifuged and stored at -80°C ready for analysis.

### 3M2H glutamine conjugate analysis

3-Methyl-2-hexanoic acid glutamine conjugate was extracted from the frozen cell pellets using 10 µl (N-methyl-N-tertbutyldimethylsilyl)trifluoroacetamide (MTBSTFA), 1% tert-butyldimethylsilane (TBDMS) and 10 µl pyridine and 1µl triethylamine. Derivatisation was carried out in the reagent on the samples. The mixture was maintained at 70°C for 1 hour. The 3M2H glutamine derivatives were analysed by gas chromatography using an Agilent Technologies 6890/5973 gas chromatography/mass selective data system and an Agilent HP5-MS column (30m x 0.25mm id x 0.25µm film) with selected ion monitoring, id indicating internal diameter. The temperature programme used was 70°C to 270°C at 10°C/min. Peak areas were calculated automatically using the in-built software. Structures were identified by mass spectrometry and retention times to known standards.

### Cholesterol and Squalene analysis

Cholesterol and squalene were extracted from the frozen cell pellets using 20 µl of chloroform. This extract was then injected directly into the GC (details as above) and analysed under full scan using an Agilent HP5-MS column (30m x 0.25mm id x 0.25µm film). Oven programme 70°C to 270°C at 10°C/min. Structures were identified by mass spectrometry and retention times to known standards.

### Short chain fatty acids

Short chain fatty acids were extracted from the frozen cell pellets using 20 µl of chloroform. This extract was then injected directly into the GC (details as above) and analysed with selected ion monitoring using an HP-Innowax column (30m x 0.25mm id x 0.25um film). Oven programme 70°C to 240°C at 5°C/min. Structures were identified by mass spectrometry and retention times to known standards.

The relative levels of the 3M2H glutamine conjugate are shown in appended Figure 8.

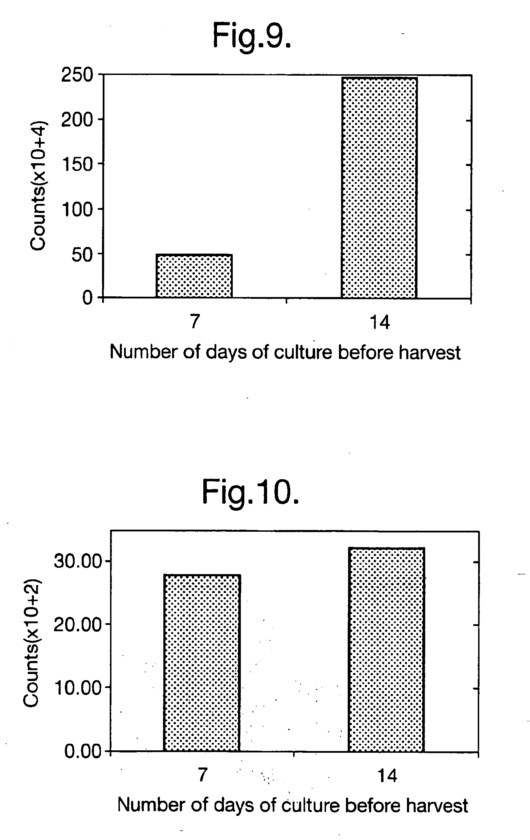

The relative levels of cholesterol in apocrine cells harvested after 7 and 14 days in culture are shown in appended Figure 9.

The relative levels of squalene in apocrine cells harvested after 7 and 14 days in culture are shown in appended Figure 10.

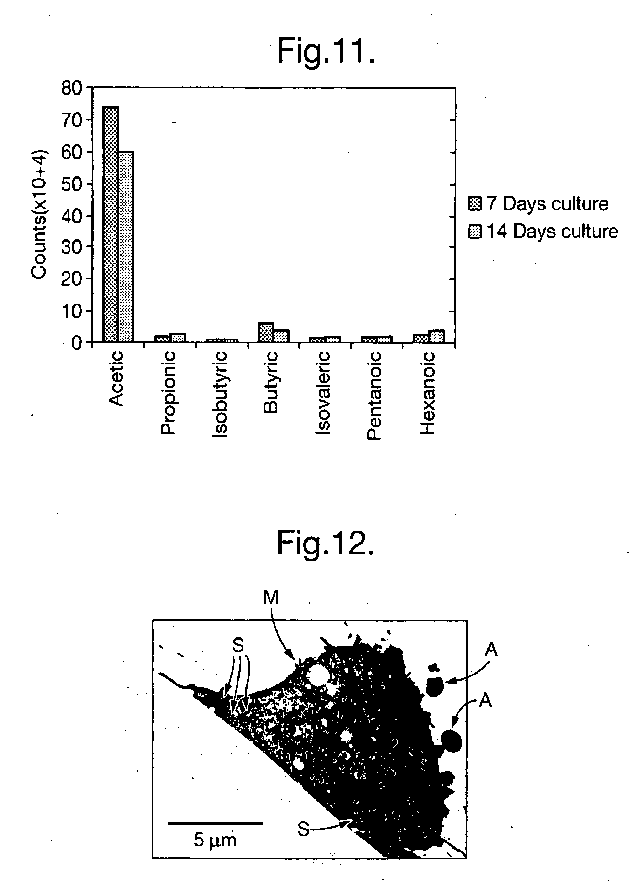

The relative levels of short chain fatty acids are shown in appended Figure 11.

C16/18 fatty acids were also detected in the 14 day sample. The data summarised herein showing the presence of various volatile fatty acids demonstrates further that the indefinitely proliferating cell line mimicked successfully the functions of an apocrine gland.

### Inhibitor studies

The production of malodour precursor compounds and the proliferation of cell growth would be expected to be modulated by the action of compounds known to interfere with androgen metabolizing enzymes. The proliferation of cells and the production of malodour precursor compounds would be compromised by the addition to the culture medium of antagonists known to interfere with androgen metabolising enzymes or with the androgen receptor directly. Such compounds would be expected to reduce the synthesis and secretion of odour precursor molecules both in culture and in apocrine glands residing in axillary skin. Such compounds include, but are not limited to:
17α-substituted benzylestradiols
Tricyclic coumarin sulfamates
Estrone-3-O-sulfamate
Tricyclic oxepin sulfamate
2-methoxyestrone-3-O-sulfamate
Substituted chromenone sulfamates
17α-benzyl (or 4'-tert-butylbenzyl)estra-1,3,5(10)-trienes
17β-(N-alkylcarbamoyl)-estra-1,3,5(10)-trien-3-O-sulfamates
Trilostane
Cyanoketone
Cyproterone acetate
Norgestrel
Norethindrone
Thiazolidinediones
16-(bromoalkyl)-estradiols
Flavonoids
Isoflavonoids
Lignans
Trifluoromethylacetylenic secoestradiol
6β-(thiaheptanamide) derivatives of estradiol
Estrone containing a spiro-gamma-lactone at position 17
**7**α-thioalkyl and **7**α-thioaryl
Derivatives of spironolactone
N-butyl-N-methyl-11-(3'-hydroxy-21', 17'-carbolactone-19'-nor-**17**'α-pregna-1', 3', 5'(10') -trien-**7**'α-yl) -undecanamide
1,4-androstadiene-1,6,17-trione
Androsterone **3**β**-**substituted derivatives
4-azasteroids (MK386)
6-azasteroidal **17**β-carboxamide triaryls
8-chloro-4-methyl-1,2,3,4,4a,5,6,10b-octaahydro-benzo[f]quinolin-3(2 H)-one (LY 191704)
6-[4-(N,N-diisopropylcarbamoyl)phenyl]-N-methyl-quinolin-2-one 5)
Benzo[c]quinolizin-3-ones
Epicatechin-3-gallate
Epigallocatechin-3-gallate
Suramin
Zinc
Azelaic acid
6-[4-(N,N-diisopropylcarbamoyl)phenyl]-1H-quinolin-2-one 4
4-[3-[5-benzyl-8-(2-methyl)propyl-10,11-dihydrodibenz[b,f]azepine-2-carboxamido]phenoxy]butyric acid
Turosteride
MK-434
Dihydrofinasteride
Chlormadinone acetate
TZP-4238
Epristeride (SK&F 105657, ONO-9302)
**17**α-estradiol
17-(5'-isoxazolyl)androsta-4,16-dien-3-one
N-(1,1,1,3,3,3-hexafluorophenyl-propyl)-3-oxo-4-aza- **5**α-androst-1-ene-**17**β-carboxamide (PNU 157706)
Dutasteride
Oxendolone (TSAA-291: **16** β-ethyl-**17**β-hydroxy-4-estren-3-one)
19-nor-10-azasteroids
Progesterone-based steroids bearing an oxime group connected to the steroidal D-ring
Benzoquinolinone
Serenoa repens extract permixon
Artocarpus incisus
Alizarin
Curcumin
Myristoleic acid
Phenazine derivatives
γ-linolenic acid
4-[3-[3-[bis(4-isobutylphenyl)-methylamino]benzoyl]-1H-indol-1-yl] butyric acid (FK 143)
Flutamide
Spironolactone

A number of compounds have been shown to reduce the proliferation of cells in the skin, including those cells originating from cutaneous appendages such as the sebaceous glands. Such compounds when contacted with the apocrine cells would be expected to reduce the synthesis and secretion of odour precursor molecules both in culture and in apocrine glands residing in axillary skin. Such compounds include, but are not limited to:
13-*cis*-retinoic acid
Retinoic acid

### Ultrastructural analysis of human apocrine cell line ASG5 by transmission electron microscopy (TEM)

### Sample preparation

ASG5 cells passage number 47 were cultured in MEGM in a 95 % air / 5 % CO₂ humidified incubator at 37 °C, as described previously. Cells were harvested after 3 and 14 days incubation, respectively. Prior to fixation cells were washed twice in phosphate buffered saline pH 7.5.

### Transmission electron microscopy

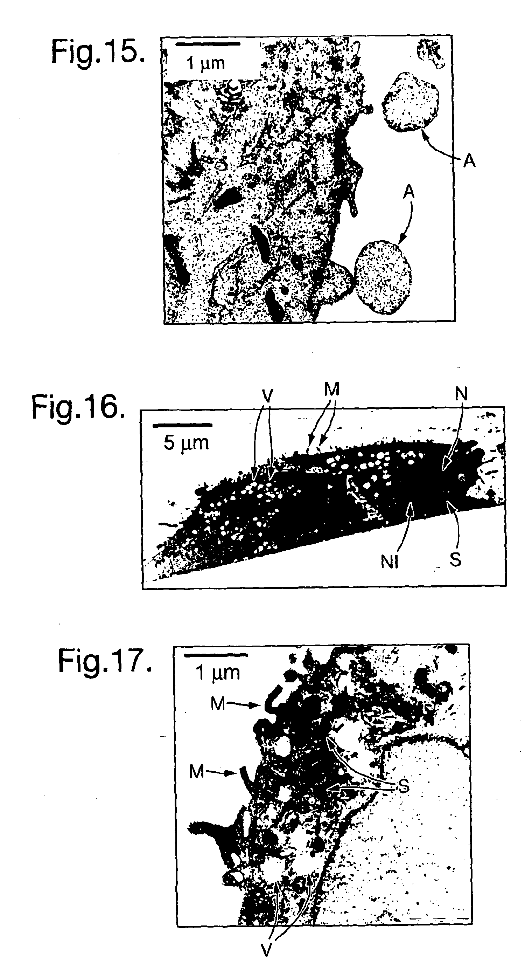

The washed cell cultures were fixed in 3 % glutaraldehyde in 0.1 M cacodylate buffer pH 7.4 for 1 hour at room temperature. The specimens were washed in 0.1 M cacodylate buffer pH 7.4 for three periods of 5 minutes. After primary fixation specimens were fixed in 1 % osmium tetroxide in 0.1 M cacodylate buffer pH 7.4 for 1 hour at room temperature. The specimens were washed again in 0.1 M cacodylate buffer pH 7.4 for three periods of 5 minutes. Specimens were then dehydrated through a grade series of ethanol. The cells were embedded in Luft's Epon resin and were polymerised at 60 °C for 48 hours. The blocks were sectioned on a Reichert "Ultracut S" Ultramicrotome set to give sections ca. 120 nm thick. The sections were picked up onto 200 mesh hexagonal thin bar copper grids. The sections were stained in uranyl acetate followed by lead citrate on a Leica EM stain. Stained sections were examined in a Philips CM120 transmission electron microscope operated at 120 kV. Images were recorded digitally, as .tif files and are shown herein as Figures 12 to 15 in respect of 3 day old culture cells and Figures 16 and 17 in respect of 14 day old culture cells.

### Results

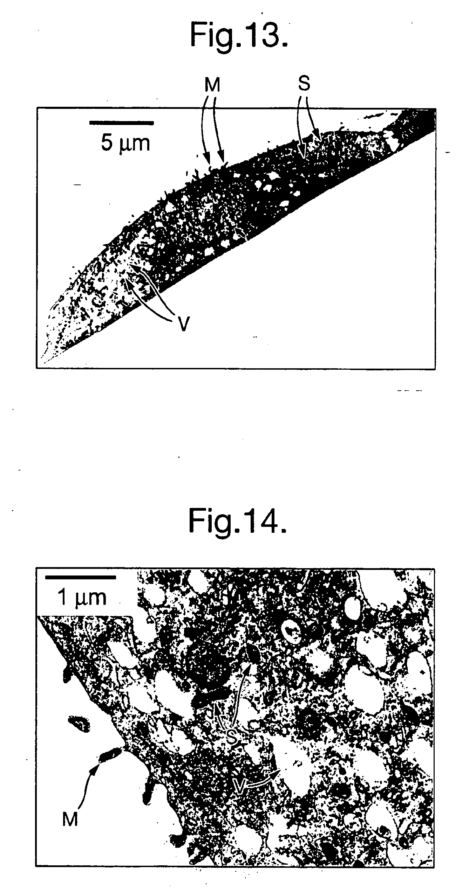

Cells shown in Figures 12, 13 and 16 exhibited a rounded or "cobblestone-like" appearance which is typical of epithelial cells in culture. The secretory nature of the cells was demonstrated by the presence of numerous microvilli identified as "M" (Figures 12, 13, 14 16 and 17) and apical blebs, designated as "A" (Figures 12, 15 and 16) at the luminal membrane, combined with the presence of numerous secretory granules, designated as "S", throughout the cell cytoplasm (Figures 12 to 17). The pinching off of small portions of apical cytoplasm i.e. the microvilli "M" and apical blebs "A" constitute the apocrine secretion process (Montagna et al. (1953) Histology and cytochemistry of human skin. V. Axillary apocrine sweat glands. Am. J. Anat., 92: 451-470). All cells contained numerous secretory granules designated as "S" (Figures 12 to 17), although it was not possible to distinguish between Type I and Type II granules in accordance with (Bell (1974) The ultrastructure of human axillary apocrine glands after epinephrine injection. J. Invest. Dermatol., 63: 147-159). The granules contained highly electron-opaque particles, presumably containing small lipid droplets. Numerous "empty" vesicles, designated as "V", were observed in most cells (Figures 12, 13, 14 16 and 17). This is probably as a consequence of the fixation process where the contents of the vesicles have been lost. Such vesicles are available to participate in the process of secretion. The cells in Figures 13 and 16 were rather flattened in appearance and clearly showed the nucleolus, designated "Nl".

In all cells from both 3 and 14 day old ASG5 cultures apical blebs, "A", luminal microvilli,"M" and numerous secretory granules, "S" were observed, confirming that these cells display typical active apocrine secretory morphology. These findings further substantiate the apocrine nature of the ASG5 cell line and provide additional evidence of the usefulness of this cell line as a functionally representative cell model of human axillary apocrine sweat glands.

## Claims

1. An apocrine cell line exhibiting long term proliferation derived from human apocrine glands, wherein long term indicates that cells retain their capability to proliferate after at least 30 sub-cultures (passages).

2. A cell line according to claim 1 that exhibits characteristic features of apocrine glands.

3. A cell line according to any preceding claim which is capable of proliferating indefinitely.

4. A cell line having the deposit number ECACC 07021301.

5. A method of obtaining a cultured apocrine cell line exhibiting long term proliferation comprising the steps of
isolating an apocrine cell from axillary skin tissue by shearing, and incubating isolated apocrine coils in a Williams E medium,
washing the incubated coils in an enzyme-free Williams E medium supplemented by epidermal growth factor, insulin hydrocortisone, holotransferrin, L-glutamine penicillin and streptomycin,
culturing the isolated cell in a first culture medium comprising mammary epithelial growth medium supplemented with epidermal growth factor, insulin hydrocortisone, bovine pituitary extract, gentamicin and amphotericin, and after 7 days culturing phorbol ester was introduced into the medium inducing premature multilayering, removing unattached cells from the first culture medium to leave a proliferative attached monolayer,
detaching and transferring said attached cells to a second culture medium comprising mammary epithelial growth medium containing an effective concentration of the phorbol ester, and thereby establishing an apocrine cell line exhibiting long-term proliferation capability.

6. Use of an apocrine cell line according to any of claims 1 to 4 or produced according to claim 5 for diagnostic, therapeutic or cosmetic preparations.

7. Use of an apocrine cell line according to any of claims 1 to 4 or produced according to claim 5 for evaluating in vitro whether a material is a deodorant when topically applied to skin.

8. Use of an apocrine cell line according to any of claims 1 to 4 or produced according to claim 5 for the examination of the physiology or the pathophysiology of human apocrine gland.

9. Use of an apocrine cell line according to any of claims 1 to 4 or produced according to claim 5 for the testing of compounds or agents against diseases.

10. Use of an apocrine cell line according to any of claims 1 to 4 or produced according to claim 5 for the preparation of products derived from said cells.

## Patentansprüche

1. Apokrine Zelllinie, die eine Langzeit-Proliferation aufweist, die aus humanen apokrinen Drüsen stammt, wobei "Langzeit-" angibt, dass Zellen ihre Fähigkeit zu proliferieren, nach wenigstens 30 Subkulturen (Passagen) beibehalten.

2. Zelllinie nach Anspruch 1, die charakteristische Merkmale von apokrinen Drüsen aufweist.

3. Zelllinie nach einem vorangehenden Anspruch, die fähig ist, unbegrenzt lange zu proliferieren.

4. Zelllinie, die die Hinterlegungsnummer ECACC 07021301 hat.

5. Verfahren zur Herstellung einer kultivierten apokrinen Zelllinie, die eine Langzeit-Proliferation aufweist, umfassend die Schritte:
Isolieren einer apokrinen Zelle aus Achselhautgewebe durch Scherung und Inkubieren der isolierten apokrinen Spulen in einem Williams E-Medium,
Waschen der inkubierten Spulen in einem enzymfreien Williams E-Medium, das mit epidermalem Wachstumsfaktor, Insulin-Hydrocortison, Holotransferrin, L-Glutamin-Penicillin und Streptomycin supplementiert ist,
Kultivieren der isolierten Zelle in einem ersten Kulturmedium, das mammäres epitheliales Wachstumsmedium, supplementiert mit epidermalem Wachstumsfaktor, Insulin-Hydrocortison, Rinderhypophysenextrakt, Gentamicin und
Amphotericin, umfasst und nach 7 Tagen Kultivieren Einführen von Phorbolester in das Medium, was vorzeitiges Multilayering induziert,
Entfernen von nicht-gebundenen Zellen von dem ersten Kulturmedium, um eine proliferative gebundene Monolayer zurückzulassen,
Ablösen und Transferieren der gebundenen Zellen in ein zweites Kulturmedium,
das mammäres epitheliales Wachstumsmedium, das eine wirksame Konzentration des Phorbolesters enthält, umfasst, und **dadurch** Entwickeln einer apokrinen Zeillinie, die Langzeit-Proliferations-Vermögen aufweist.

6. Verwendung einer apokrinen Zelllinie nach einem der Ansprüche 1 bis 4 oder hergestellt nach Anspruch 5 für diagnostische, therapeutische oder kosmetische Zubereitungen.

7. Verwendung einer apokrinen Zelllinie nach einem der Ansprüche 1 bis 4 oder hergestellt nach Anspruch 5 zur in vitro-Evaluierung, ob ein Material ein Deodorant ist, wenn es topisch auf die Haut aufgetragen wird.

8. Verwendung einer apokrinen Zelllinie nach einem der Ansprüche 1 bis 4 oder hergestellt nach Anspruch 5 für die Untersuchung der Physiologie oder der Pathophysiologie einer humanen apokrinen Drüse.

9. Verwendung einer apokrinen Zelllinie nach einem der Ansprüche 1 bis 4 oder hergestellt nach Anspruch 5 für das Testen von Verbindungen oder Mitteln gegen Erkrankungen.

10. Verwendung einer apokrinen Zelllinie nach einem der Ansprüche 1 bis 4 oder hergestellt nach Anspruch 5 für die Herstellung von Produkten, die von den genannten Zellen stammen.

## Revendications

1. Lignée de cellules apocrines présentant une prolifération de longue durée et dérivant de glandes apocrines humaines, dans laquelle une longue durée indique que les cellules conservent leur capacité de prolifération après au moins 30 sous-cultures (passages).

2. Lignée de cellules selon la revendication 1, présentant des caractéristiques de glandes apocrines.

3. Lignée de cellules selon l'une quelconque des revendications précédentes, qui est capable de proliférer indéfiniment.

4. Lignée de cellules ayant le numéro de dépôt ECACC 07021301.

5. Méthode d'obtention d'une lignée de cellules apocrines en culture présentant une prolifération de longue durée, comprenant les étapes suivantes :
■ l'isolement d'une cellule apocrine provenant d'un tissu cutané axillaire par cisaillement et l'incubation des glomerules apocrines ("apocrine coils") isolées dans un milieu E de Williams,
■ le lavage des cellules ("coils") incubées dans un milieu E de Williams exempt d'enzyme et complété avec le facteur de croissance de l'épiderme ("EGF"), de l'insuline, de l'hydrocortisone, de l'holotransferrine, de la L-glutamine, de la pénicilline et de la streptomycine,
■ la culture de la cellule isolée dans un premier milieu de culture comprenant un milieu de croissance de l'épithélium mammaire ("MEGM") complété avec le facteur de croissance de l'épiderme ("EGF"), de l'insuline, de l'hydrocortisone, un extrait d'hypophyse de bovin, de la gentamicine et de l'amphotéricine, et après 7 jours de culture, un ester de phorbol est introduit dans le milieu pour induire la formation prématurée de plusieurs couches,
■ l'élimination des cellules non attachées du premier milieu de culture pour laisser une monocouche attaché en prolifération,
■ le détachement et le transfert desdites cellules attachées vers un second milieu de culture comprenant un milieu de croissance de l'épithélium mammaire contenant une concentration efficace de l'ester de phorbol, pour ainsi établir une lignée de cellules apocrines présentant une capacité de prolifération de longue durée.

6. Utilisation d'une lignée de cellules apocrines selon l'une quelconque des revendications 1 à 4 ou produite selon la revendication 5, pour des préparations diagnostiques, thérapeutiques ou cosmétiques.

7. Utilisation d'une lignée de cellules apocrines selon l'une quelconque des revendications 1 à 4 ou produite selon la revendication 5, pour évaluer *in vitro* si une substance est un déodorant quand elle est appliquée sur la peau par voie topique.

8. Utilisation d'une lignée de cellules apocrines selon l'une quelconque des revendications 1 à 4 ou produite selon la revendication 5, pour l'examen de la physiologie ou de la pathophysiologie de la glande apocrine humaine.

9. Utilisation d'une lignée de cellules apocrines selon l'une quelconque des revendications 1 à 4 ou produite selon la revendication 5, pour tester des composés ou des agents contre des maladies.

10. Utilisation d'une lignée de cellules apocrines selon l'une quelconque des revendications 1 à 4 ou produite selon la revendication 5, pour la préparation de produits dérivant desdites cellules.
